(19) Europäisches Patentamt
European Patent Office
Office européen des brevets

(11) **EP 2 024 300 B1**

(12) **EUROPÄISCHE PATENTSCHRIFT**

(45) Veröffentlichungstag und Bekanntmachung des Hinweises auf die Patenterteilung:
**13.03.2013 Patentblatt 2013/11**

(21) Anmeldenummer: **07725213.8**

(22) Anmeldetag: **15.05.2007**

(51) Int Cl.:
*C04B 35/624* (2006.01)    *A61K 6/02* (2006.01)
*A61K 6/08* (2006.01)    *A61C 13/00* (2006.01)
*C04B 35/80* (2006.01)    *C04B 35/185* (2006.01)
*C04B 35/83* (2006.01)    *A61K 6/00* (2006.01)
*C04B 35/18* (2006.01)

(86) Internationale Anmeldenummer:
**PCT/EP2007/004294**

(87) Internationale Veröffentlichungsnummer:
**WO 2007/137696 (06.12.2007 Gazette 2007/49)**

(54) **VERFAHREN ZUR HERSTELLUNG EINER KERAMIK**

METHOD FOR PRODUCING A CERAMIC

PROCÉDÉ DE FABRICATION D'UNE CÉRAMIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.05.2006 DE 102006024489**

(43) Veröffentlichungstag der Anmeldung:
**18.02.2009 Patentblatt 2009/08**

(73) Patentinhaber: **Karlsruher Institut für Technologie**
**76131 Karlsruhe (DE)**

(72) Erfinder:
• **BINDER, Joachim**
**76227 Karlsruhe (DE)**
• **HAUSSELT, Jürgen**
**76726 Germersheim (DE)**
• **RITZHAUPT-KLEISSL, Hans-Joachim**
**69190 Walldorf (DE)**
• **SCHLECHTRIEMEN, Nadja**
**76351 Linkenheim-Hochstetten (DE)**

(74) Vertreter: **Gärtner, Stephan et al**
**Karlsruher Institut für Technologie**
**Stabsabteilung Innovation**
**Postfach 36 40**
**76021 Karlsruhe (DE)**

(56) Entgegenhaltungen:
**EP-A- 0 328 041    EP-A- 1 435 346**
**EP-A- 1 818 318    WO-A-02/20425**
**WO-A-90/11979    WO-A-02/074714**
**DE-A1- 19 938 143    US-A- 5 185 177**
**US-A1- 2002 006 532**

## Beschreibung

Die Erfindung betrifft ein Verfahren zu Herstellung einer Keramik.

In der Dentaltechnik werden sowohl glaskeramische als auch oxidkeramische Werkstoffe zur Herstellung von vollkeramischem festsitzendem Zahnersatz eingesetzt. Während Glaskeramiken über ein schmelztechnologisches Verfahren hergestellt werden, ist für oxidkeramische Werkstoffe ein pulvertechnologisches Verfahren erforderlich.

Da bei dem natürlichen Erscheinungsbild eines Zahnes die Intensität der Farbwirkung und der Trübung von der Schneide zum Zahnhals hin steigt, werden glaskeramische Verblendkeramiken in unterschiedlichen Farbintensitäten und Trübungen hergestellt. Diese unterschiedlichen Massen werden vom Zahntechniker zu einem mehrschichtigen Aufbau auf einer unifarbenen Gerüststruktur z.B. aus Oxidkeramik verwendet, um somit die Form und Farbe der natürlichen Zähne zu imitieren.

Im Bereich der Glaskeramiken gibt es ein Verfahren zur Herstellung eines mehrfarbigen vollkeramischen Zahnersatzes mit Hilfe der CAD/CAM Technologie. In der Firmenschrift *Machinable Ceramics* (2005) der Fa. VITA Zahnfabrik H. Rauter GmbH & Co. KG, Bad Säckingen, wird die Glaskeramik *VITABLOCS*® *TriLuxe* vorgestellt, die eine Farbabstufung mit drei Farben aufweist. Es handelt sich um Glaskeramikblöcke aus einer Feinstruktur-Feldspatkeramik mit drei verschiedenen, in einen Block integrierten Farbsättigungsgraden. Diese Glaskeramikblöcke werden im dichten Zustand zum Zahnersatz geschliffen, so dass weitere thermische Behandlungen zur Sinterung bei Einsatz dieses Materials nicht erforderlich sind.

Oxidkeramische Werkstoffe erfordern aufgrund ihrer spezifischen Eigenschaften, z.B. ihrer hohen Schmelzpunkte, andere Formgebungsverfahren als die in der Zahntechnik verwendeten Glaskeramiken. Diese Oxidkeramiken sind weiß und können mit verschiedenen Techniken auf zahnfarbene Grundtöne monochromatisch eingefärbt werden. Hierzu wird der Zahnersatz aus oxidkeramischen Pulvern entweder geformt, angesintert und mit einer Glasschmelze infiltriert oder im angesinterten Zustand geformt, anschließend mit einer speziellen Farbflüssigkeit infiltriert und abschließend dicht gesintert. Durch die Infiltration der eingefärbten Glasschmelze bzw. der Farbflüssigkeit in das poröse Gerüst ist eine farbliche Anpassung möglich, wobei jedoch das Gerüst stets einfarbig bleibt.

Die DE 10 2004 045 752 B3 beschreibt eine Keramik, die form- und dimensionstreu sintert und im gesinterten Zustand unifarben weiß erscheint. Weiterhin wird hier beschrieben, dass eine Einfärbung des weißen Grundmaterials durch Zugabe von Pigmenten und Metalloxiden auf dentale Grundfarben möglich ist. Die Verarbeitung dieses Materials erfolgt, indem aus einem pulvertechnologisch hergestellten zylindrischen Grünkörper mit Hilfe der CAD/CAM Technologie die gewünschte Form spanabhebend herausgearbeitet wird.

Beim Einsatz von zwei unterschiedlich eingefärbten pulvertechnologisch hergestellten Keramiken kann das Schwindungsverhalten während der Sinterung sehr unterschiedlich sein, so dass sich die Keramiken nicht ohne weiteres formtreu sintern lassen. Zudem ändern sich beim Einsatz von Farbpigmenten weitere Eigenschaften der Keramik, wie z.B. deren Sinterverhalten als Funktion der Sintertemperatur und der Haltezeit.

Die DE 199 44 130 A1 offenbart ein Verfahren zur Herstellung einer farbigen Dentalkeramik durch subtraktive Bearbeitung von speziell angefertigten Materialblöcken, die Gradienten in Bezug auf Farbe, Transluzenz, Helligkeit bzw. Transparenz aufweisen. Hierzu wird in einem CAD-System die Position des Zahnersatzes im Materialblock solange verändert, bis die optischen Eigenschaften des Zahnersatzes möglichst optimal sind.

Die DE 197 14 178 A1 beschreibt ein Verfahren zur Herstellung eines mehrfarbigen Formkörpers für die Weiterverarbeitung zu einer Zahnrestauration, worin mindestens zwei unterschiedliche gefärbte Ausgangsmaterialien in eine Pressmatrize eingefüllt und zum Formkörper verpresst werden. Der fertig erstellte und ggf. temperatur- und/oder strahlenbehandelte Formkörper wird anschließend konfektioniert und zu einer Zahnrestauration weiterverarbeitet.

Ausgehend hiervon ist es die Aufgabe der vorliegenden Erfindung, ein Verfahren zu Herstellung einer Keramik, mit diesem Verfahren hergestellte Keramiken und ihre Verwendung vorzuschlagen, die die genannten Nachteile und Einschränkungen nicht aufweisen.

Ein derartiges Verfahren soll die Herstellung von pulvertechnologisch hergestelltem Zahnersatz, insbesondere Kronen, mit mindestens zwei Farbtönen oder einem Farbgradienten erlauben, ohne dass die Formtreue der so hergestellten Keramik beeinträchtigt wird.

Diese Aufgabe wird im Hinblick auf das Verfahren durch die Schritte des Anspruchs 1 gelöst. Die Unteransprüche beschreiben jeweils vorteilhafte Ausgestaltungen der Erfindung.

Ein für das erfindungsgemäße Verfahren geeigneter Grünkörper besteht aus zwei, drei, vier, fünf, sechs oder mehr verschiedenen Pulvermischungen, die mit einem Druck von bis zu 600 MPa zu einem Formkörper verdichtet wurden. Um eine gute Formtreue der hiermit hergestellten Keramik zu erhalten, wird isostatisches Pressen bevorzugt. Als Pulvermischungen eignen sich insbesondere Granulate, die zusätzlich Additive, z.B. Binder oder Presshilfsmittel, umfassen.

Jedes dieser Pulvermischungen (Granulate) enthält jeweils

- ein keramisches Pulver, vorzugsweise yttriumstabilisiertes $ZrO_2$, und

- färbende Metallverbindungen, bevorzugt einen Anteil von bis zu 10 Gew.%, besonders bevorzugt bis zu 5 Gew.%, insbesondere Oxide von Übergangselementen, vorzugsweise von Cu, Cr, Mn, Fe, Co, Ni, V, Nd oder Pr, und/oder Farbpigmente, bevorzugt einen Anteil von bis zu 20 Gew.%, besonders bevorzugt bis zu 10 Gew.%, insbesondere auf Zirkon- oder Baddeleyit-Basis.

In einer besonderen Ausgestaltung enthält eine Pulvermischung (Granulat) darüber hinaus ein metallisches und/oder ein intermetallisches Pulver, bevorzugt ZrSi$_2$, insbesondere bei gleichzeitiger Anwesenheit von ZrO$_2$ als keramisches Pulver.

Die verschiedenen Pulvermischungen (Granulate) in einem derartigen Grünkörper unterscheiden sich in ihrer Zusammensetzung (Gehalt) an den färbenden Metallverbindungen und/oder Farbpigmenten.

In einer bevorzugten Ausgestaltung sind zwei oder mehr Pulvermischungen (Granulate) im Grünkörper in Schichten übereinander angeordnet.

Enthält der Grünkörper drei oder mehr verschiedene, jeweils übereinander angeordnete Schichten von Granulaten, so weisen die Granulate einer Schicht in einer besonderen Ausgestaltung jeweils einen höheren Gehalt an färbenden Metallverbindungen und/oder Farbpigmenten im Vergleich zu einer der benachbarten Schichten und einen geringeren Gehalt an färbenden Metallverbindungen und/oder Farbpigmenten im Vergleich zur anderen der benachbarten Schichten auf. Die Schichten können hierbei durch Mischungen aus zwei Granulaten mit jeweils verschiedenen Anteilen bestehen.

In einer weiteren Ausgestaltung wurden die zwei oder mehr Pulvermischungen (Granulate) derart in den Grünkörper eingebracht, dass der Grünkörper einen Farbgradienten besitzt. Dies kann pulvertechnologisch z. B. so erfolgen, dass anfangs nur ein erstes Pulver zugegeben wird, dem nach und nach ein stetig höherer Anteil an einem zweiten Pulver zugemischt wird.

Zur erfindungsgemäßen Herstellung der Keramik wird jeweils ein derartiger Grünkörper bereitgestellt und vorzugsweise bei einer Temperatur von 1100 °C bis 1700 °C, besonders bevorzugt von 1350 °C bis 1550 °C, gesintert.

Die Formgebung der Keramik erfolgt vor der Sinterung und/oder als Zwischenschritt, während dessen die Sinterung unterbrochen und nach der Formgebung weitergeführt wird.

In einer besonders bevorzugten Ausgestaltung werden die Zusammensetzungen der einzelnen Pulvermischungen (Granulate) so gewählt, dass das Sintern des Formkörpers formtreu erfolgt.

In einer weiteren Ausgestaltung werden die Zusammensetzungen der einzelnen Pulvermischungen (Granulate) so gewählt, dass das Sintern des Formkörpers schwindungsfrei erfolgt.

Erfindungsgemäße Keramiken eigenen sich insbesondere als farblich gradierte Dentalkeramiken (Rohlinge), die als vollkeramischer Zahnersatz dienen.

Die vorliegende Erfindung löst somit das Problem der Formgebung und der Sinterung mit zwei oder mehr unterschiedlichen Pulvermischungen (Granulaten). Prinzipielle Voraussetzung für die Herstellung von passgenauem Zahnersatz ist, dass die Pulvermischungen während der Sinterung gleiche oder zumindest sehr ähnliche Volumenänderungen aufweisen. Werden z. B. zwei verschiedene Pulvermischungen eingesetzt, ist es vor allem wichtig, dass die Volumenänderungen $\Delta V$ der eingesetzten zu einem Grünkörper verdichteten Pulvermischungen am Ende des Herstellungsverfahrens identisch sind:

$$\Delta \widetilde{V}_1 = \Delta \widetilde{V}_2 \qquad\qquad (1)$$

Hierbei gilt:

$$\Delta \widetilde{V}_1 = (1 + \Delta \widetilde{m}_1) \frac{\rho_{\text{Grünkörper, 1}}}{\rho_{\text{Sinterkörper, 1}}} \qquad\qquad (2a)$$

$$\Delta \widetilde{V}_2 = (1 + \Delta \widetilde{m}_2) \frac{\rho_{\text{Grünkörper, 2}}}{\rho_{\text{Sinterkörper, 2}}} \qquad\qquad (2b)$$

wobei, $\Delta \widetilde{m}$ die jeweilige Massenänderung der Pulvermischungen und $\rho$ die jeweils angegebene Dichte bezeichnen.

Werden die Gleichungen 2a und 2b in Gleichung 1 eingesetzt, folgt, dass für die Passgenauigkeit am Ende des Verfahrens folgende Bedingung erfüllt sein muss:

$$(1 + \Delta\tilde{m}_1)\frac{\rho_{\text{Grünkörper, 1}}}{\rho_{\text{Sinterkörper, 1}}} = (1 + \Delta\tilde{m}_2)\frac{\rho_{\text{Grünkörper, 2}}}{\rho_{\text{Sinterkörper, 2}}} \qquad (3a)$$

oder umgeformt

$$\frac{(1 + \Delta\tilde{m}_1)}{(1 + \Delta\tilde{m}_2)} \cdot \frac{\rho_{\text{Grünkörper, 1}}}{\rho_{\text{Grünkörper, 2}}} = \frac{\rho_{\text{Sinterkörper, 1}}}{\rho_{\text{Sinterkörper, 2}}} \qquad (3b)$$

Aus Gleichung 3b wird ersichtlich, dass die Verarbeitungseigenschaften der Pulvermischungen bei der entsprechenden Formgebung genauso wichtig sind wie das Sinterverhalten und die Zusammensetzung der Pulvermischungen.

Die erste Lösung für das Problem der Herstellung formtreu sinternder farblich gradierter Keramiken ergibt sich, wenn die Pulvermischungen mit unterschiedlichen Farbtönen nach der Herstellung der Formkörper identische Gründichten aufweisen sowie nach dem thermischen Prozess identische Sinterdichten. Während des Prozesses müssen zudem gleiche Massenänderungen auftreten. Dies ergibt sich dann, wenn die Hauptkomponenten identisch sind und bereits geringe Zusätze zu den gewünschten Farbänderungen führen.

In manchen Fällen genügen geringe Zusätze vor allem von Farbpigmenten jedoch nicht, um die gewünschte Farbeinstellung zu erzielen, insbesondere bei opaken Keramiken. Zudem können in einigen Fällen selbst geringe Mengen an Zusätzen das Sinterverhalten derart ändern, dass entweder die Formtreue am Ende des Prozesses nicht mehr gegeben ist oder die Unterschiede im Sinterverhalten bereits während der Herstellung zu Spannungen und Rissen in der Keramik führen. Dieser Effekt ist umso ausgeprägter, je mehr Farbpigment zugegeben wird, wobei auch die Zusammensetzung des Pigments und die Aufbereitung einen Einfluss ausüben können. Unterschiedliches Sinterverhalten führt letztendlich zu einer anisotropen Schwindung und somit zu einem Verzug.

Eine zweite Lösung für das Problem der Herstellung formtreu sinternder farblich gradierter Keramiken liegt daher in der genauen Abstimmung der Komponenten der einzelnen Pulvermischungen. Je nach Wahl der Komponenten ergibt sich eine unterschiedliche Massenänderung. Große Differenzen führen zu Verzug und ermöglichen daher keine formtreue Herstellung von farblich gradierten Keramiken.

Die Abstimmung der Zusammensetzung der Pulvermischungen in einem System aus verschiedenen Pulvermischungen ist möglich, wenn eine Komponente im System über einen weiten Bereich keinen Einfluss auf Farbe hat und die Massenänderungen und das geänderte Sinterverhalten angepasst werden können. Durch Variation des Anteils dieser *inerten* Komponente lässt sich ein Grünkörper mit zwei oder mehreren unterschiedlichen Pulvermischungen (Granulaten) bereitstellen, der formtreu sintert und zu einer farblich gradierten Keramik führt. Dies ist vor allem bei der Verwendung von metallischen und intermetallischen Verbindungen zielführend.

Die Erfindung wird anhand der folgenden Ausführungsbeispiele und des Vergleichsbeispiels näher erläutert.

**Ausführungsbeispiel 1:**

Im Wirbelschichtgranulator wurden die Granulate A, B und C eines yttriumstabilisierten Zirkoniumoxids mit verdünnten, wässrigen Lösungen unterschiedlicher Konzentration an Färbeflüssigkeit im Verhältnis Granulat : Lösung = 2,5 : 1 gemäß Tabelle 1 beschichtet:

| Tabelle 1 | |
|---|---|
| Granulat | Konzentration der Färbeflüssigkeit im Wasser [Gew.%] |
| A | 0,0 |
| B | 10,0 |
| c | 15,0 |

Die Beschichtung führte bei den bei 1450 °C gesinterten Formkörpern A', B' und C' der Granulate A, B bzw. C zu deutlich erkennbaren Farbunterschieden.

Die Charakterisierung der Farbe erfolgt nach dem CIE-L*a*b*-System der Commission Internationale de l'Eclairage (CIE), Wien, 1986. In dieser Systematik beschreiben die Parameter

L* die Helligkeitsempfindung,

a* die Rot-/Grün-Empfindung, wobei a* > 0 eine rote und a* < 0 eine grüne Empfindung und

b* die Gelb-/Blau-Empfindung, wobei b* > 0 eine gelbe und

b* < 0 eine blaue Empfindung

jeweils der Netzhaut des menschlichen Auges bedeutet.

In Tabelle 2 sind die experimentell ermittelten Farbunterschiede der Sinterkörper A', B' und C' als L*a*b*-Werte (jeweils Mittelwerte aus 5 Messungen) angegeben:

| Tabelle 2 | | | |
|---|---|---|---|
| Sinterkörper | L*-Wert | a*-Wert | b*-Wert |
| A' | 88,27 | 0,16 | 2,13 |
| B' | 85,34 | -0,84 | 11,36 |
| C' | 69,23 | 4,67 | 19,75 |

Zur Herstellung eines zweifach geschichteten zylindrischen Formkörpers wurden ca. 5 g des Granulats A in eine Pressmatrize gegeben und hierauf ca. 5 g des Granulats B geschüttet.

Anschließend wurden die betreffenden Granulate axial zu einem zylindrischen Formkörper verdichtet und bei ca. 400 MPa kaltisostatisch verdichtet.

Aus diesen Formkörpern wurden gut zu vermessende Formkörper gefräst und sowohl vor als auch nach dem Sintern bei 1450 °C vermessen. Die Formkörper bestanden im oberen Teil aus Granulat A und im unteren Teil aus Granulat B. In Tabelle 3 sind jeweils die Mittelwerte der Längen und Breiten von zweifach geschichteten Formkörpern vor und nach dem Sintern aufgeführt:

| Tabelle 3: Zweifach geschichteter Formkörper | | | |
|---|---|---|---|
| | Vor dem Sintern | Nach dem Sintern | Differenz |
| Länge oben | 12,031 mm | 9,787 mm | - 18,7 % |
| Länge unten | 12,050 mm | 9,805 mm | - 18,6 % |
| Breite oben | 7,943 mm | 6,467 mm | - 18,6 % |
| Breite unten | 7,949 mm | 6,468 mm | - 18,6 % |

Die gemessenen Längenänderungen lagen in einem Bereich von 18,6 - 18,7 %, d.h. es trat ein Sinterschwund auf. Der Formkörper wies jedoch vor und nach dem Sintern die gleiche Form auf, da die sich die Längenänderungen höchstens um 0,1 % unterschieden. Es lag also eine formtreue Sinterung vor.

**Vergleichsbeispiel:**

Zwei Granulate D und E wurden aus $ZrSi_2$, $ZrO_2$, MgO, $Al_2O_3$, einem siliziumhaltigen Polymer sowie einem Presshilfsmittel hergestellt. Granulat E enthielt darüber hinaus 7 Gew.% eines Farbpigments auf Baddeleyit-Basis. Die Zugabe des Pigmentes auf Baddeleyit-Basis beim Granulat E führte bei den bei 1500 °C gesinterten Körpern D', E' der Granulate D, E zu leichten Farbunterschieden, die in Tabelle 4 als L*a*b*-Werte (jeweils Mittelwerte aus 5 Einzelmessungen) angegeben sind:

| Tabelle 4: | | | |
|---|---|---|---|
| Sinterkörper | L*-Wert | a*-Wert | b*-Wert |
| D' | 97,03 | -1,02 | 2,91 |
| E' | 91,22 | -1,50 | 6,27 |

Ca. 5 g des Granulats D wurden in eine Pressmatrize gegeben und ca. weitere 5 g des Granulats E wurden auf Granulat D gegeben. Die beiden Granulate wurden anschließend axial zu einem Formkörper verpresst und in einer kaltisostatischen Presse bei 350 MPa verdichtet. Aus den zylindrischen Formkörpern wurden gut zu vermessende Formkörper gefräst und sowohl vor als auch nach dem Sintern vermessen.

Die Formkörper bestanden im unteren Teil aus Granulat D und im oberen Teil aus Granulat E. Die derart gefrästen Formkörper wurden anschlie-βend bei 1500°C gesintert. In Tabelle 5 sind jeweils die Mittelwerte der Längen und Breiten von drei zweifach geschichteten Formkörpern vor und nach dem Sintern angegeben:

| Tabelle 5: Zweifach geschichteter Formkörper | | | |
|---|---|---|---|
| | Vor dem Sintern | Nach dem Sintern | Differenz |
| Länge oben | 12,098 mm | 11,936 mm | - 1,3 % |
| Länge unten | 12,124 mm | 12,491 mm | + 3,0 % |
| Breite oben | 8,035 mm | 7,933 mm | - 1,3 % |
| Breite unten | 8,049 mm | 8,262 mm | + 2,7 % |

Während Granulat E bereits bei 1500 °C dicht sinterte ($p$ = 4,2 g/cm$^3$), wurde bei Granulat D bei dieser Temperatur lediglich eine Dichte von 3,7 g/cm$^3$ erreicht. Dadurch ergab sich eine um ca. 4 % höhere Längenänderung, was sich in einem deutlichen Verzug der Formkörper widerspiegelte.

**Ausführungsbeispiel 2**

Zwei Granulate F und G wurden aus $ZrSi_2$, $ZrO_2$, MgO, $Al_2O_3$, einem siliziumhaltigen Polymer, einem Presshilfsmittel sowie mindestens 1. Gew.% eines Mineralisierers hergestellt. Granulat F enthielt darüber hinaus bis zu 7 Gew.% eines Farbpigments auf Baddeleyit-Basis. Zur Einfärbung auf dentale Grundfarben wurde den Granulaten F und G gemäß Tabelle 6 unterschiedliche Mengen an Mischungen färbender Metalloxide zugegeben, die $MnO_2$, $Pr_6O_{11}$, $Fe_2O_3$ und CuO enthielten:

| Tabelle 6: | | |
|---|---|---|
| Granulat | F | G |
| $ZrSi_2$ [Gew.%] | 47,9 | 48,0 |
| $ZrO_2$ (monoklin) [Gew.%] | 27,3 | 32,0 |
| $ZrO_2$ (Farbpigment auf Baddelyit Basis) [Gew.%] | 7,0 | 0,0 |
| $Al_2O_3$ [Gew.%] | 7,0 | 7,0 |
| MgO [Gew.%] | 0,5 | 0,5 |
| Mineralisierer [Gew.%] | 0,5 | 0,5 |
| Färbende Metalloxidmischung [Gew.%] | 1,9 | 4,1 |
| Siliziumhaltiges Polymer [Gew.%] | 5,9 | 5,9 |
| Presshilfsmittel [Gew.%] | 2,0 | 2,0 |

Die in Tabelle 6 aufgeführten Zusammensetzungen führten bei den bei 1450 °C dicht gesinterten Körpern F', G' der Granulate F, G zu deutlich erkennbaren Farbunterschieden, die in Tabelle 7 als L*a*b*-Werte (jeweils Mittelwerte aus 5 Einzelmessungen) angegeben sind:

| Tabelle 7: | | | |
|---|---|---|---|
| Sinterkörper | L*-Wert | a*-Wert | b*-Wert |
| F' | 80,54 | 0,72 | 14,94 |
| G' | 69,88 | 3,88 | 22,68 |

Zur Herstellung eines zweifach geschichteten zylindrischen Formkörpers wurden ca. 5 g des Granulats F in eine Pressmatrize gegeben und hierauf ca. 5 g des Granulats G geschüttet.

Zur Herstellung eines fünffach geschichteten zylindrischen Formkörpers wurden aus den Granulaten F und G jeweils ca. 2 g Mischungen mit den prozentualen Anteilen aus Tabelle 8 hergestellt und übereinander in eine Pressmatrize gegeben:

| Tabelle 8: | | |
|---|---|---|
| Granulat | F | G |
| 1. Schicht [Gew.%] | 100 | 0 |
| 2. Schicht [Gew.%] | 75 | 25 |
| 3. Schicht [Gew.%] | 50 | 50 |
| 4. Schicht [Gew.%] | 25 | 75 |
| 5. Schicht [Gew.%] | 0 | 100 |

Anschließend wurden die betreffenden Granulate jeweils axial zu einem zylindrischen Formkörper verdichtet und bei ca. 350 MPa kaltisostatisch verdichtet.

Aus diesen Formkörpern wurden gut zu vermessende Formkörper gefräst und sowohl vor als auch nach dem Sintern bei 1450 °C vermessen. Die Formkörper bestanden im oberen Teil aus Granulat F und im unteren Teil aus Granulat G. In Tabelle 9a und 9b sind jeweils die Mittelwerte der Längen und Breiten von zwei zweifach bzw. fünffach geschichteten Formkörpern vor und nach dem Sintern aufgeführt:

| Tabelle 9a: Zweifach geschichteter Formkörper | | | |
|---|---|---|---|
| | Vor dem Sintern | Nach dem Sintern | Differenz |
| Länge oben | 12,041 mm | 12,053 mm | 0,1 % |
| Länge unten | 12,059 mm | 12,042 mm | - 0,1 % |
| Breite oben | 7,977 mm | 7,977 mm | 0,0 % |
| Breite unten | 7,974 mm | 7,966 mm | -0,1 % |

| Tabelle 9b: Fünffach geschichteter Formkörper | | | |
|---|---|---|---|
| | Vor dem Sintern | Nach dem Sintern | Differenz |
| Länge oben | 12,038 mm | 12,050 mm | 0,1 % |
| Länge unten | 12,078 mm | 12,074 mm | < 0,1 % |
| Breite oben | 7,981 mm | 7,989 mm | 0,1 % |
| Breite unten | 7,992 mm | 7,982 mm | -0,1 % |

Die gemessenen Längenänderungen lagen in einem Bereich von ± 0,1%, d.h. es trat kein Sinterschwund auf. Da der Formkörper sowohl vor als auch nach dem Sintern die gleiche Form aufwies, erfolgte die Sinterung formtreu und schwindungsfrei.

**Patentansprüche**

1. Verfahren zur Herstellung einer Keramik, wobei zunächst ein Grünkörper, der aus mindestens zwei verschiedenen, zu einem Formkörper verdichteten Pulvermischungen besteht, die jeweils ein keramisches Pulver sowie eine färbende Metallverbindung und/oder ein Farbpigment enthalten, wobei sich jeweils zwei Pulvermischungen in ihrer Zusammensetzung an färbender Metallverbindung und/oder Farbpigment unterscheiden, hergestellt und anschließend gesintert wird, wobei die Formgebung der Keramik vor der Sinterung und/oder als Zwischenschritt, während dessen die Sinterung unterbrochen und nach der Formgebung weitergeführt wird, erfolgt und wobei die mindestens zwei Pulvermischungen während der Sinterung gleiche Volumenänderungen aufweisen.

2. Verfahren nach Anspruch 1, wobei die Zusammensetzung der mindestens zwei Pulvermischungen so gewählt wird, dass eine Pulvermischung über einen weiten Bereich keinen Einfluss auf die Farbe der Keramik hat.

3. Verfahren nach Anspruch 1 oder 2, wobei Granulate als Pulvermischungen eingesetzt werden.

4. Verfahren nach einem der Ansprüche 1 bis 3, wobei yttriumstabilisiertes $ZrO_2$ als keramisches Pulver eingesetzt wird.

5. Verfahren nach einem der Ansprüche 1 bis 4, wobei ferner ein metallisches und/oder intermetallisches Pulver eingesetzt wird.

6. Verfahren nach einem der Ansprüche 1 bis 5, wobei Pulvermischungen, die bis zu 10 Gew.% färbende Metallverbindungen und/oder bis zu 20 Gew.% Farbpigmente enthalten, eingesetzt werden.

7. Verfahren nach einem der Ansprüche 1 bis 6, wobei die mindestens zwei Pulvermischungen im Grünkörper in Schichten übereinander angeordnet werden.

8. Verfahren nach Anspruch 7, wobei mindestens drei Pulvermischungen im Grünkörper in Schichten derart übereinander angeordnet werden, dass die Pulvermischungen einer Schicht jeweils einen höheren Gehalt an färbenden Metallverbindungen und/oder Farbpigmenten im Vergleich zu einer der benachbarten Schichten und jeweils einen niedrigeren Gehalt an färbenden Metallverbindungen und/oder Farbpigmenten im Vergleich zur anderen der benachbarten Schichten aufweisen.

9. Verfahren nach einem der Ansprüche 1 bis 6, wobei die Pulvermischungen im Grünkörper derart gemischt werden, dass der Grünkörper einen stetigen Farbgradienten aufweist.

## Claims

1. Method for producing a ceramic, wherein a green body, which consists of at least two different powder mixtures that are compacted to form a moulded body, said powder mixtures respectively containing a ceramic powder and a colouring metal compound and/or a colour pigment, wherein each set of two powder mixtures differs in its composition of colouring metal compound and/or colour pigment, is initially produced and subsequently sintered, wherein the ceramics are shaped prior to sintering and/or as an intermediate step, during which sintering is interrupted and then continued after shaping and wherein the at least two powder mixtures have the same volume changes during sintering.

2. Method according to claim 1, wherein the composition of the at least two powder mixtures is selected such that a powder mixture, over a wide range, has no influence on the colour of the ceramics.

3. Method according to claim 1 or 2, wherein granulates are used as powder mixtures.

4. Method according to any one of the preceding claims 1 to 3, wherein yttrium-stabilised $ZrO_2$ is used as ceramic powder.

5. Method according to any one of the preceding claims 1 to 4, wherein a metallic and/or intermetallic powder is also used.

6. Method according to any on of the preceding claims 1 to 5, wherein powder mixtures are used which contain up to 10% by weight of colouring metal compounds and/or up to 20% by weight of colour pigments.

7. Method according to any one of the preceding claims 1 to 6, wherein the at least two powder mixtures in the green body are arranged in layers on top of each other.

8. Method according to claim 7, wherein at least three powder mixtures in the green body are arranged in layers on top of each other such that the powder mixtures of a layer each have a higher content of colouring metal compounds and/or colour pigments compared to one of the adjacent layers and each have a lower content of colouring metal compounds and/or colour pigments compared to the other one of the adjacent layers.

9. Method according to any one of the preceding claims 1 to 6, wherein the powder mixtures in the green body are mixed such that the green body has a steady colour gradient.

**Revendications**

1. Procédé d'obtention d'une céramique selon lequel on fabrique tout d'abord une ébauche constituée d'au moins deux mélanges pulvérulents, différents, comprimés en un corps moulé, qui renferment respectivement une poudre céramique, ainsi qu'un composé métallique de coloration et/ou un pigment coloré, deux mélanges pulvérulents se distinguant respectivement par leur composition en composé métallique de coloration et/ou en pigment coloré, puis, on effectue un frittage de cette ébauche, la mise en forme de la céramique étant effectuée avant le frittage et/ou en tant qu'étape intermédiaire au cours de laquelle le frittage est interrompu puis poursuivi après la mise en forme, et les deux mélanges pulvérulents présentant les mêmes modifications de volume perdant le frittage.

2. Procédé conforme à la revendication 1,
selon lequel
la composition des deux mélanges pulvérulents est choisie de sorte qu'un mélange de poudre dans une large plage n'ait aucune influence sur la couleur de la céramique.

3. Procédé conforme à la revendication 1 ou 2,
selon lequel
on met en oeuvre des granulés en tant que mélange pulvérulent.

4. Procédé conforme à l'une des revendications 1 à 3,
selon lequel
on met en oeuvre du $ZrO_2$ stabilisé par de l'yttrium en tant que poudre céramique.

5. Procédé conforme à l'une des revendications 1 à 4,
selon lequel
on met en outre en oeuvre une poudre métallique et/ou intermétallique.

6. Procédé conforme à l'une des revendications 1 à 5,
selon lequel
on met en oeuvre des mélanges pulvérulents qui renferment jusqu'à 10 % en poids de composés métalliques de coloration et/ou jusqu'à 20 % en poids de pigments colorés.

7. Procédé conforme à l'une des revendications 1 à 6,
selon lequel
les deux mélanges pulvérulents sont disposés l'un sur l'autre en couches dans l'ébauche.

8. Procédé conforme à la revendication 7,
selon lequel
on dispose au moins trois mélanges pulvérulents en couche dans l'ébauche, de sorte que les mélanges pulvérulents d'une couche, aient respectivement une teneur en composés métalliques de coloration et/ou en pigments colorés supérieure à celle de l'une des couches voisines, et respectivement une teneur en composés métalliques de coloration et/ou en pigments colorés inférieure à celle de l'autre couche voisine.

9. Procédé conforme à l'une des revendications 1 à 6,
selon lequel
les mélanges pulvérulents sont mélangés dans l'ébauche, de sorte que celle-ci présente toujours un gradient de couleur.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004045752 B3
- DE 19944130 A1
- DE 19714178 A1